# EUROPEAN PATENT SPECIFICATION

(11) **EP 0 665 887 B1**
(45) Date of publication and mention of the grant of the patent: **14.10.1998**
(21) Application number: 94923813.3
(22) Date of filing: 18.08.1994
(51) Int. Cl.: C12N 15/12, C12N 15/11, C07K 14/705, C12P 21/08, G01N 33/53, A61K 39/395, A61K 31/40

(54) **HUMAN 5-HT 2? RECEPTOR**
HUMANE 5-HT2 REZEPTOR
RECEPTEUR HUMAIN DE 5-HT 2?

(30) Priority: 20.08.1993 GB 9317349; 14.01.1994 GB 9400597
(43) Date of publication of application: 09.08.1995
(73) Proprietor: SMITHKLINE BEECHAM PLC, Brentford, Middlesex TW8 9EP (GB)
(72) Inventor: CAREY, Janet, Elizabeth, The Pinnacles,Harlow,Essex CM29 5AD (GB); FLANIGAN, Thomas, Patrick, Woodstock Road Oxford OX2 6HE (GB)
(74) Representative: Valentine, Jill Barbara
(86) International application number: GB9401813
(87) International publication number: WO9506117

(56) References cited:
- WO-A-89/08149
- WO-A-93/11147
- BIOCHEMICAL AND BIOPHYSICAL RESEARCH COMMUNICATIONS, vol.181, no.3, 31 December 1991, DULUTH, MINNESOTA US pages 1469 - 1478 SALTZMAN ET AL. 'Cloning of the human serotonin 5-HT2 and 5-HT1C receptor subtypes'

## Description

This invention relates to a novel member of the 5-HT₂ subclass of serotonin receptors, isolated nucleic acids encoding same, recombinant host cell transformed with serotonin receptor encoding DNA and to uses of the expressed receptor and nucleic acid sequences in drug screening and development as well as in therapeutic and diagnostic applications. More specifically the invention relates to a novel human 5-HT₂ receptor.

5-Hydroxytryptamine (also variously referred to as 5-HT, 5HT, or serotonin) is a potent, naturally occurring neurotransmitter which exerts its effects via cell surface receptors found on serotonergic neurons. At least four major families of 5-HT receptor are known (5-HT₁₋₄). Each family is comprised of one or more types denominated, for example, 5-HT₁ₐ, 5-HT_{1b}, 5-HT_{1c}, 5-HT_{1d}, or 5-HT₁ₑ. Further, within certain 5-HT receptors types various subtypes are appreciated, such as 5-HT_{1dα} or 5-HT_{1dβ}. Although reasonably certain, it is fair to say that the nomenclature in this field is in a state of flux and revision as new receptors are identified or as new properties are ascribed to old receptors. The nomenclature in this field has been recently reviewed by P.P.A. Humphrey *et al.,* "A proposed new nomenclature for 5-HT receptors" 1993 Trends Pharmacol. Sci. vol,14 pp.233-236, the contents of which are incorporated herein by reference for purposes of background. Briefly, a receptor is assigned to a particular family, type or subtype based on its shared molecular biological, pharmacological and biochemical properties with other members of that family, type or subtype. There is also a generalized grouping for receptors that for one reason or another have not yet been characterized sufficiently to permit their unequivocal assignment to a recognized class. These are the so-called "orphan" receptors.

A particularly useful technique, known as the rapid filtration radioligand assay permits the characterization of receptors based on their relative affinities for various radioactively labeled ligands. For example, most members of the 5-HT₁ family of receptors display less than 10 nM affinity for 5-HT and 5-carboxyaminotryptamine (5-CT);10-1000 nM affinity for methysergide, methiothepin, quipazine, and m-trifluoromethylphenylpiperazine (TFMPP) and greater than 1000 nM affinity for 3-alpha-tropanyl-1H-indole-3-carboxylic acid ester (ICS 205-930) and 1-alphaH, 3-alpha, 5-alphaH-tropan 3yl-3,5-dichlorobenzoate (MDL 72222). Currently, the 5-HT₁ family includes the a, b, d and e types. The 5-HT_{1c} type receptor, has been renamed 5-H_{2c} and is characterized as a member of the 5-HT₂ family along with the 5-HT₂ₐ and _{2b} types on the basis of structural homology, pharmacology and stimulus transduction pathways.

Based on molecular biological properties certain members of families can be arranged into so-called super families. For example the 5-HT_{2c, 2a, 2b, 1dα, 1dβ} and ₁ₑ receptors all belong to a super family of receptors having seven putative transmembrane domains and are G protein coupled.

The rat stomach fundus preparation has been used as an *in vitro* bioassay for serotonin (5-HT) for a considerable time. The fundus receptor has high affinity for 5-HT, rawolscine and yohimbine and is insensitive to spiperone and ketanserin. Recent molecular cloning stategies have resulted in the isolation, expression, and pharmacological characterisation of the 'rat stomach fundus receptor' (Foguet *et al.* EMBO J. 11(9) 3481-3487; Kursar *et al.,* 1992 Mol. Pharmacol. 42, 549-557) and indicate that this 5-HT receptor consititutes a third member of the 5-HT_{1c/2} receptor subfamily. This receptor has therefore been provisionally classified as the 5-HT_{2b} sub-type. That this receptor is not species-specific is highlighted by Loric *et al.,* 1992 FEBS Letters 312(2,3) 203-207) who have isolated the mouse homologue of this receptor; its pharmacological profile is consistent with it being a member of the 5-HT₂ receptor sub-family. Foguet *et al.,* (1992) and Kursar *et al.,* (1992) did not find evidence for the expression of the rat fundus receptor outside the stomach fundus, but Loric *et al.,* (1992) used the polymerase chain reaction (PCR) to demonstrate the presence of receptor mRNA in mouse intestine, heart, kidney and brain. This suggests a more widespread distribution (at least in mouse) of this receptor than previously thought While species differences may account for the discrepancies seen in receptor mRNA distribution in these studies, differences in the sensitivities of the analytical techniques used may provide a more likely explanation. Indeed, detection of 'fundic' 5-HT receptor mRNA outside the stomach fundus requires amplification techniques such as nested PCR, which suggests that a low abundance of this transcript occurs in these tissues. Alternatively, these data may reflect a highly regionalised localisation of this receptor mRNA to specific cell types. Foguet *et al.,* (1992, above), comment that, as determined by PCR amplification of genomic DNA, the fundus 5-HT receptor gene is present in the human genome.

This invention provides a novel human receptor of the 5-HT₂ type. The DNAs of this invention, such as the specific sequences disclosed herein, are useful in that they encode the genetic information required for the expression of this novel subtype of receptor. Additionally, the sequences may be used as probes in order to isolate and identify additional members of the family, type and subtype as well as forming the basis of antisense therapy for disease conditions which are characterized by an atypical expression of the novel gene. The novel protein is itself useful as a therapeutic or diagnostic agent as well as a component in a screening system for drugs which are antagonists or agonists of the receptor activity. These and additional uses for the reagents described herein will be come apparent to those of ordinary skill in the art upon reading this specification.

Samples of post-mortem human cortex, hippocampus and amygdala (Dr. B. MacDonald, Dept. of Neuropathology, Radcliffe Infirmary, Oxford) were used to prepare poly (A)⁺ mRNA for use in RT-PCR with pairs of oligonucleotides derived from the rat fundus 5-HT receptor nucleotide sequence (Foguet *et al.,* 1992). With each sample the major RT-PCR product obtained exhibited a size equivalent to that which was predicted from the rat (and mouse) nucleotide sequence(s). Thus the human homologue of the rat fundus 5-HT receptor is believed to be transcriptionally functional in cortex, hippocampus and amygdala as well as in the stomach and small intestine.

This invention provides isolated nucleic acid molecules encoding a novel human 5HT₂ receptor, including mRNAs, DNAs, cDNAs as well as antisense analogs thereof and biologically active and diagnostically or therapeutically useful fragments thereof.

In particular, the invention provides an isolated nucleic acid molecule selected from the group consisting of:
(a) a nucleic acid molecule comprising the DNA sequence shown in Seq. ID. No. 1 or Seq. ID No. 3 and
(b) a nucleic acid molecule differing from the nucleic acid molecule of (a) in codon sequence due to the degeneracy of the genetic code but encoding the same protein.

This invention also provides recombinant vectors, such as cloning and expression plasmids useful as reagents in the recombinant production of the novel 5-HT₂ proteins or peptides, as well as recombinant prokaryotic and/or eukaryotic host cells comprising novel 5-HT₂ nucleic acid sequence.

This invention also provides methods of identifying ligands capable of binding to the novel 5-HT₂ receptor by measuring the binding of the ligand to be identified relative to known ligands.

Methods for screening drugs to identify those which interact with and bind to the novel 5-HT₂ receptors may employ the receptors in isolated form in solution or in immobilized form or expressed on the surface of recombinant host cells. Regardless of the form of the receptor, a plurality of candidate drugs are contacted with the receptor under conditions sufficient to form a drug/receptor binding complex and drugs capable of forming, enhancing or interfering with said complexes are detected.

Nucleic acid probes comprise nucleic acid molecules of sufficient length to specifically hybridize to the novel 5-HT₂ sequences.

This invention also provides an antisense oligonucleotide having a sequence capable of binding with mRNAs encoding the novel 5-HT₂ receptors so as to prevent the translation of said mRNA.

Transgenic non-human animals comprise a nucleic acid molecule encoding a novel 5-HT₂ receptor. Said transgenic animals may be used as models for differential receptor expression, mutation and SAR (structure/activity relationship) evaluation as well as in ligand and drug screens.

This invention also provides fusion proteins comprising a novel 5-HT₂ receptor domain and a receptor/ligand binding indicator domain capable of providing an analytically detectable signal. Also provided are methods of screening drugs by forming, enhancing or interfering with the detectable signal.

This invention also provides a method of screening compounds to identify those compounds which bind to a novel 5-HT₂ receptor comprising: providing a recombinant host cell expressing on the surface thereof a novel 5-HT₂ receptor, said receptor being associated with a second component capable of providing a detectable signal in response to the binding of a compound to said receptor; contacting a plurality of candidate compounds with said host cells under conditions sufficient to permit binding of compounds to the receptor; and identifying those compounds capable of receptor binding by detecting the signal produced by said second component.

This invention further provides a new human 5-HT₂ receptor.

In further describing the present invention, the following additional terms will be employed, and are intended to be defined as indicated below.

"Fusion protein" is a protein resulting from the expression of at least two operatively-linked heterologous coding sequences. The protein comprising a 5-HT₂ protein or fragment thereof and a second unrelated peptide sequence is an example of a fusion protein.

A coding sequence is "operably linked to" another coding sequence when RNA polymerase will transcribe the two coding sequences into a single mRNA, which is then translated into a single polypeptide having amino acids derived from both coding sequences. The coding sequences need not be contiguous to one another so long as the expressed sequence is ultimately processed to produce the desire protein.

"Recombinant" polypeptides refer to polypeptides produced by recombinant DNA techniques; i.e., produced from cells transformed by an exogenous DNA construct encoding the desired polypeptide. "Synthetic" polypeptides are those prepared by chemical synthesis.

A "replicon" is any genetic element (e.g., plasmid, chromosome, virus) that functions as an autonomous unit of DNA replication *in vivo;* i.e., capable of replication under its own control.

A "vector" is a replicon, such as a plasmid, phage, or cosmid, to which another DNA segment may be attached so as to bring about the replication of the attached segment.

A "double-stranded DNA molecule" refers to the polymeric form of deoxyribonucleotides (bases adenine, guanine, thymine, or cytosine) in a double-stranded helix, both relaxed and supercoiled. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the sense strand of DNA.

A DNA "coding sequence of' or a "nucleotide sequence encoding" a particular protein, is a DNA sequence which is transcribed and translated into a polypeptide, when placed under the control of appropriate regulatory sequences.

A "promoter sequence" is a DNA regulatory region capable of binding RNA polymerase in a cell and initiating transcription of a downstream (3' direction) coding sequence. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes..

DNA "control sequences" refers collectively to promoter sequences, ribosome binding sites, polyadenylation signals, transcription termination sequences, upstream regulatory domains, enhancers, and the like, which collectively provide for the expression (i.e., the transcription and translation) of a coding sequence in a host cell.

A control sequence "directs the expression" of a coding sequence in a cell when RNA polymerase will bind the promoter sequence and transcribe the coding sequence into mRNA, which is then translated into the polypeptide encoded by the coding sequence.

A "host cell" is a cell which has been transformed or transfected, or is capable of transformation or transfection by an exogenous DNA sequence.

A cell has been "transformed" by exogenous DNA when such exogenous DNA has been introduced inside the cell membrane. Exogenous DNA may or may not be integrated (covalently linked) into chromosomal DNA making up the genome of the cell. In prokaryotes and yeasts, for example, the exogenous DNA may be maintained on an episomal element, such as a plasmid. With respect to eukaryotic cells, a stably transformed or transfected cell is one in which the exogenous DNA has become integrated into the chromosome so that it is inherited by daughter cells through chromosome replication. This stability is demonstrated by the ability of the eukaryotic cell to establish cell lines or clones comprised of a population of daughter cells containing the exogenous DNA.

A "clone" is a population of cells derived from a single cell or common ancestor by mitosis. A "cell line" is a clone of a primary cell that is capable of stable growth *in vitro* for many generations.

Two DNA or polypeptide sequences are "substantially homologous" or "substantially the same" when at least about 85% (preferably at least about 90%, and most preferably at least about 95%) of the nucleotides or amino acids match over a defined length of the molecule. As used herein, substantially homologous also refers to sequences showing identity to the specified DNA or polypeptide sequence. DNA sequences that are substantially homologous can be identified in a Southern hybridization experiment under, for example, stringent conditions, as defined for that particular system. Defining appropriate hybridization conditions is within the skill of the art. See, e.g., "Current Protocols in Mol. Biol." Vol. I & II, Wiley Interscience. Ausbel *et al.* (ed.) (1992). Protein sequences that are substantially the same can be identified by proteolytic digestion, gel electrophoresis and microsequencing.

The term "functionally equivalent" intends that the amino acid sequence of the subject protein is one that will elicit a serotonergic response, as defined above, equivalent to the specified 5-HT₂ peptide.

A "heterologous" region of a DNA construct is an identifiable segment of DNA within or attached to another DNA molecule that is not found in association with the other molecule in nature. Thus, when the heterologous region encodes a receptor gene, the gene will usually be flanked by DNA that does not flank the gene in the genome of the source animal. Another example of a heterologous coding sequence is a construct where the coding sequence itself is not found in nature (e.g., synthetic sequences having codons different from the native gene). Allelic variation or naturally occurring mutational events do not give rise to a heterologous region of DNA, as used herein.

This invention provides an isolated nucleic acid molecule encoding the novel human 5-HT₂ receptor. Such a receptor is defined by the criteria discussed above. One means for isolating a novel human 5-HT₂ receptor coding nucleic acid is to probe a human genomic or cDNA library with a natural or artificially designed probe using art recognized procedures (See for example: "Current Protocols in Molecular Biology", Ausubel, F.M., et al. (eds.) Greene Publishing Assoc. and John Wiley Interscience, NewYork, 1989,1992). One particularly useful probe for this purpose is a probe incorporating all, or a hybridizable fragment, of a DNA of the sequences disclosed herein as Seq. ID. Nos. 1 and 3. The isolated nucleic acid molecules obtained hereby may be used to obtain complementary copies of genomic DNA, cDNA or RNA from human, mammalian or other animal sources or to screen such sources for related sequences including transcriptional regulatory and control elements defined above as well as other stability, processing, translation and tissue specificity-determining regions from 5' and/or 3' regions relative to the coding sequences disclosed herein. Additional coding sequences isolated by the procedures disclosed herein are considered to be substantially the same as the coding sequence given in Seq. ID. Nos.1 and 3 if the additional sequence share at least about 85% homology with the sequence of Seq. ID. Nos. 1 or 3.

This invention also provides for an isolated protein which is a novel human 5-HT₂ receptor defined by the amino acid sequence listed in Seq. ID. No. 2 or 4 or variants with a substantially homologous amino acid sequence having at least an 85% match with, but retaining the receptor binding activity of, the novel 5-HT₂ receptor.

In particular, the invention provides a novel 5HT₂ receptor comprising the amino acid sequence listed in Seq.ID. No. 2 or having the amino acid sequence listed in Seq. ID. No. 4.

The invention further provides isolated nucleic acid molecules encoding the novel 5-HT₂ receptor of the invention.

The proteins of this invention are preferably made by recombinant genetic engineering techniques. The invention therefore provides a process for preparing a novel 5-HT₂ receptor which comprises expressing DNA encoding said receptor and recovering the expression product.

The isolated nucleic acids particularly the DNAs can be introduced into expression vectors by operatively linking the DNA to the necessary expression control regions (e.g. regulatory regions) required for gene expression. The vectors can be introduced into the appropriate host cells such as prokaryotic (e.g., bacterial), or eukaryotic (e.g. yeast, insect or mammalian) cells by methods well known in the art (Ausubel et *al.,supra).* The coding sequences for the desired proteins having been prepared or isolated, can be cloned into a suitable vector or replicon. Numerous cloning vectors are known to those of skill in the art, and the selection of an appropriate cloning vector is a matter of choice. Examples of recombinant DNA vectors for cloning and host cells which they can transform include the bacteriophage λ (*E*. *coli),* pBR322 (*E*. *coli),* pACYC177 (*E*. *coli),* pKT230 (gram-negative bacteria), pGV1106 (gram-negative bacteria), pLAFR1 (gram-negative bacteria), pME290 (non-*E*. *coli* gram-negative bacteria), pHV14 (*E*. *coli* and *Bacillus subtilis),* pBD9 *(Bacillus),* pIJ61 *(Streptomyces),* pUC6 *(Streptomyces),* YIp5 *(Saccharomyces),* a baculovirus insect cell system, , YCp19 (Saccharomyces). See, generally, "DNA Cloning": Vols. I & II, Glover *et al.* ed. IRL Press Oxford (1985) (1987) and; T. Maniatis *et al.* ("Molecular Cloning" Cold Spring Harbor Laboratory (1982).

The gene can be placed under the control of a promoter, ribosome binding site (for bacterial expression) and, optionally, an operator (collectively referred to herein as "control" elements), so that the DNA sequence encoding the desired protein is transcribed into RNA in the host cell transformed by a vector containing this expression construction. The coding sequence may or may not contain a signal peptide or leader sequence. The proteins of the present invention can be expressed using, for example, the *E*. *coli* tac promoter or the protein A gene (spa) promoter and signal sequence. Leader sequences can be removed by the bacterial host in post-translational processing. See, e.g., U.S. Patent Nos. 4,431,739; 4,425,437; 4,338,397.

In addition to control sequences, it may be desirable to add regulatory sequences which allow for regulation of the expression of the protein sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences, the positioning and orientation of the coding sequence with respect to the control sequences being such that the coding sequence is transcribed under the "control" of the control sequences (i.e., RNA polymerase which binds to the DNA molecule at the control sequences transcribes the coding sequence). Modification of the coding sequences may be desirable to achieve this end. For example, in some cases it may be necessary to modify the sequence so that it may be attached to the control sequences with the appropriate orientation; i.e., to maintain the reading frame. The control sequences and other regulatory sequences may be ligated to the coding sequence prior to insertion into a vector, such as the cloning vectors described above. Alternatively, the coding sequence can be cloned directly into an expression vector which already contains the control sequences and an appropriate restriction site. Modification of the coding sequences may also be performed to alter codon usage to suit the chosen host cell, for enhanced expression

In some cases, it may be desirable to add sequences which cause the secretion of the polypeptide from the host organism, with subsequent cleavage of the secretory signal. It may also be desirable to produce mutants or analogs of the receptors of interest. Mutants or analogs may be prepared by the deletion of a portion of the sequence encoding the protein, by insertion of a sequence, and/or by substitution of one or more nucleotides within the sequence. Techniques for modifying nucleotide sequences, such as site-directed mutagenesis, are well known to those skilled in the art. See, e.g., T. Maniatis et al., supra; DNA Cloning, Vols. I and II, supra; Nucleic Acid Hybridization, supra.

A number of prokaryotic expression vectors are known in the art. See, e.g., U.S. Patent Nos. 4,578,355; 4,440,859; 4,436,815; 4,431,740; 4,431,739; 4,428,941; 4,425,437; 4,418,149; 4,411,994; 4,366,246; 4,342,832; see also U.K. Patent Applications GB 2,121,054; GB 2,008,123; GB 2,007,675; and European Patent Application 103,395. Yeast expression vectors are also known in the art. See, e.g., U.S. Patent Nos. 4,446,235; 4,443,539; 4,430,428; see also European Patent Applications 103,409; 100,561; 96,491. pSV2neo (as described in J. Mol. Appl. Genet, 1:327-341) which uses the SV40 late promoter to drive expression in mammalian cells or pCDNA1neo or pCDNA3, vectors derived from pCDNA1(Mol. Cell Biol. 7:4125-29) which uses the CMV promoter to drive expression. Both these latter two vectors can be employed for transient or stable(using G418 resistance) expression in mammalian cells. Preferably a vector is employed which uses the CMV promoter and encodes a neomycin resistance gene to select for transfectants on the basis of ability to grow in G418 and a dihydrofolate reductase gene which permits amplification of the transfected gene in DHFR⁻ cells. Insect cell expression systems, e.g., *Drosophila* and *Spodoptera,* are also useful, see for example, PCT applications WO 90/06358 and WO 92/06212 as well as EP 290,261-B1.

Depending on the expression system and host selected, the proteins of the present invention are produced by growing host cells transformed by an expression vector described above under conditions whereby the protein of interest is expressed. The protein is then isolated from the host cells and purified. If the expression system secretes the protein into growth media, the protein can be purified directly from the media. If the protein is not secreted, it is isolated from cell lysates or recovered from the cell membrane fraction. In the case, as here, where the protein is localized to the cell surface, whole cells or isolated membranes can be used as an assayable source of the desired gene product. The selection of the appropriate growth conditions and recovery methods are within the skill of the art.

An alternative method to identify proteins of the present invention is by constructing gene libraries, using the resulting clones to transform *E. coli* and pooling and screening individual colonies using polyclonal serum or monoclonal antibodies to the desired receptor

The proteins of the present invention may also be produced by chemical synthesis such as solid phase peptide synthesis, using known amino acid sequences or amino acid sequences derived from the DNA sequence of the genes of interest. Such methods are known to those skilled in the art. Chemical synthesis of peptides is not particularly preferred.

The proteins of the present invention or their fragments comprising at least one epitope can be used to produce antibodies, both polyclonal and monoclonal. If polyclonal antibodies are desired, a selected mammal, (e.g., mouse, rabbit, goat, horse, etc.) is immunized with a receptor of the present invention, or its fragment, or a mutated receptor. Serum from the immunized animal is collected and treated according to known procedures. If serum containing polyclonal antibodies is used, the polyclonal antibodies can be purified by immunoaffinity chromatography or other known procedures.

Monoclonal antibodies to the proteins of the present invention, and to the fragments thereof, can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. See, e.g., M. Schreier *et al.,* "Hybridoma Techniques" (1980); Hammerling *et al.,* "Monoclonal Antibodies and T-cell Hybridomas" (1981); Kennett *et al.,* "Monoclonal Antibodies" (1980); see also U.S. Patent Nos. 4,341,761; 4,399,121; 4,427,783; 4,444,887; 4,452,570; 4,466,917; 4,472,500; 4,491,632; and 4,493,890. Panels of monoclonal antibodies produced against the antigen of interest, or fragment thereof, can be screened for various properties; i.e., for isotype, epitope, affinity, etc. Monoclonal antibodies are useful in purification, using immunoaffinity techniques, of the individual antigens which they are directed against. Alternatively, genes encoding the monoclonals of interest may be isolated from the hybridomas by PCR techniques known in the art and cloned and expressed in the appropriate vectors. The antibodies of this invention, whether polyclonal or monoclonal have additional utility in that they may be employed reagents in immunoassays, RIA, ELISA, and the like.

This invention provides a method for determining whether a ligand previously not known to bind to the novel human 5-HT₂ receptor can bind to such a receptor comprising contacting the ligand to be identified with a cell comprising the coding sequence of the novel human 5-HT₂ receptor and expressing same on its surface under conditions sufficient for binding of ligands previously identified as binding to such a receptor. In other embodiments cell membrane fractions comprising the receptor or isolated receptors free or immobilized on solid supports may be used to measure binding of the ligand to be tested. When recombinant cells are used for purposes of expression of the receptor it is preferred to use cells with little or no endogenous receptor activity so that binding if any is due to the presence of the expressed receptor of interest. Preferred cells include human embryonic kidney cells (HEK-293cells), monkey kidney, fibroblast (COS) cells, Chinese hamster ovary (CHO) cells, *Drosophila* or murine L-cells. It is also preferred to employ as a host cell, one in which a receptor responsive second messenger system exists. Well known second messenger systems include but are not limited to increases or decreases in phosphoinositide hydrolysis, adenylate cyclase, guanylate cyclase, or ion channel activity in response to ligand binding to extracellular receptor domains. In a further embodiment a specifically designed indicator of receptor binding can be constructed. For example a fusion protein can be made by fusing the receptor of this invention with a protein domain which is sensitive to receptor ligand binding. Such a domain referred to here as an indicator domain is capable, itself, or in association with accessory molecules, of generating an analytically detectable signal which is indicative of receptor ligand binding.

Alternatively, cell membrane preparations from transfected or transformed cells may be employed. In such a case binding of an analytically detectable ligand is measured. The use of radioactively and non-radioactively labeled ligands is contemplated by this invention. All of the above techniques that are useful for ligand identification are also useful in drug screening and drug development protocols.

In the compound screening embodiment of this invention, the novel human 5-HT₂ receptor in isolated, immobilized or cell bound form is contacted with a plurality of candidate molecules and those candidates are selected which bind to and interact with the receptor. The binding or interaction can be measured directly by using radioactively labeled candidate of interest or by the second messenger effect resulting from the interaction or binding of the candidate compound. Alternatively, the candidate compounds can be subjected to a competition screening assays, in which a known ligand, preferably labeled with an analytically detectable reagent, most preferably radioactivity, is introduced with the drug to be tested and the compound's capacity to inhibit or enhance the binding of the labeled ligand is measured. compounds are screened for their increased affinity and selectivity to the receptor class of interest.

This invention also contemplates pharmaceutical compositions comprising a compound when identified by the above methods, such as an agonist or antagonist, and a pharmaceutically acceptable carrier. Pharmaceutical compositions of proteineous drugs of this invention are particularly useful for parenteral administration, i.e., subcutaneously, intramuscularly or intravenously. The compositions for parenteral administration will commonly comprise a solution of the compounds of the invention or a cocktail thereof dissolved in an acceptable carrier, preferably an aqueous carrier. A variety of aqueous carriers may be employed, e.g., water, buffered water, 0.4% saline, 0.3% glycine, and the like. These solutions are sterile and generally free of particulate matter. These solutions may be sterilized by conventional, well known sterilization techniques. The compositions may contain pharmaceutically acceptable auxiliary substances as required to approximate physiological conditions such as pH adjusting and buffering agents, etc. The concentration of the compound of the invention in such pharmaceutical formulation can very widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 ml sterile buffered water, and 50 mg of a compound of the invention. Similarly, a pharmaceutical composition of the invention for intravenous infusion could be made up to contain 250 ml of sterile Ringer's solution, and 150 mg of a compound of the invention. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania.

The compounds described herein can be lyophilized for storage and reconstituted in a suitable carrier prior to use. This technique has been shown to be effective with conventional proteins and art-known lyophilization and reconstitution techniques can be employed.

In situations where the identified drug is non-proteineous, it may be administered alone or in combination with pharmaceutically acceptable carriers. The proportion of which is determined by the solubility and chemical nature of the compound, chosen route of administration and standard pharmaceutical practice. For example, they may be administered orally in the form of tablets or capsules containing such excepients as starch, milk sugar, certain types of clay and so forth. They may be administered sublingually in the form of troches or lozenges in which the active ingredient is mixed with sugar and corn syrups, flavoring agents and dyes; and then dehydrated sufficiently to make it suitable for pressing into a solid form. They may be administered orally in the form of solutions which may be injected parenterally, that is, intramuscularly, intravenously or subcutaneously. For parenteral administration they may be used in the form of a sterile solution containing other solutes, for example, enough saline or glucose to make the solution isotonic.

The physician will determine the dosage of the present therapeutic agents which will be most suitable and it will vary with the form of administration and the particular compound chosen, and furthermore, it will vary with the particular patient under treatment. He will generally wish to initiate treatment with small dosages substantially less than the optimum dose of the compound and increase the dosage by small increments until the optimum effect under the circumstances is reached. It will generally be found that when the composition is administered orally, larger quantities of the active agent will be required to produce the same effect as a smaller quantity given parenterally. The compounds are useful in the same manner as other serotonergic agents and the dosage level is of the same order of magnitude as is generally employed with these other therapeutic agents. The therapeutic dosage will generally be from 1 to 10 milligrams per day and higher although it may be administered in several different dosage units. Tablets containing from 0.5 to 10 mg. of active agent are particularly useful.

Depending on the patient condition, the pharmaceutical compositioncomprising an antagonist or agonist can be administered for prophylactic and/or therapeutic treatments of disease states associated with excess or insufficient 5-HT₂ receptor activation, respectively. In therapeutic application, compositions are administered to a patient already suffering from a disease in an amount sufficient to cure or at least partially arrest the disease and its complications. In prophylactic applications, compositions containing the present compounds or a cocktail thereof are administered to a patient not already in a disease state to enhance the patient's resistance.

Single or multiple administrations of the pharmaceutical compositions can be carried out with dose levels and pattern being selected by the treating physician. In any event, the pharmaceutical composition of the invention should provide a quantity of the compounds of the invention sufficient to effectively treat the patient.

The nucleic acid embodiment of this invention is particularly useful in providing probes capable of specific hybridization with the novel human 5-HT₂ sequences. Probing technology is well known in the art and it is appreciated that the size of the probes can vary widely but it is preferred that the probe be at least 15 nucleotides in length. It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. This invention contemplates, for example using receptor encoding probes in the diagnostic evaluation of disease states characterized by an abnormal, if increased or decreased level of receptor gene expression. Alternatively, the probes can be used to identify individuals carrying chromosomal or molecular mutations in the gene encoding the receptor. Depending on the conditions employed by the ordinary skilled artisan, the probes can be used to identify and recover additional examples of this receptor from other cell types and individuals. As a general rule the more stringent the hybridization conditions the more closely related genes will be that are recovered.

Also within the scope of this invention are antisense oligonucleotides predicated upon the sequences disclosed herein for the novel 5-HT₂ receptor. Synthetic oligonucleotides or related antisense chemical structural analogs are designed to recognize and specifically bind to a target nucleic acid encoding the receptor gene and inhibit gene expression, e.g., the translation of the gene when the target nucleic acid is mRNA. Although not wishing to be bound to a particular theory for the mechanism of action of antisense drugs, it is believed that such drugs can act by one or more of the following mechanisms: by binding to mRNA and inducing degradation by endogenous nucleases such as RNase I or by inhibiting the translation of mRNA by binding to regulatory factors or ribosomal components necessary for productive protein synthesis. Additionally the antisense sequences can be use as components of a complex macromolecular arrays in which the sequences are combined with ribozyme sequences or reactive chemical groups and are used to specifically target mRNAs of interest and degrade or chemically modify said mRNAs. The general field of antisense technology is illustrated by the following disclosures which are incorporated herein by reference for purposes of background (Cohen, J.S., Trends in Pharm. Sci. 10:435(1989) and Weintraub, H.M. Scientific American Jan.(1990) at page 40).

This invention also contemplates antibodies, monoclonal or polyclonal directed to epitopes corresponding to amino acid sequences disclosed herein from the novel 5-HT₂ receptor. Particularly important regions of the receptor for immunological purposes are those hydrophilic region associated with extracellular domains of the receptor. Antibodies directed to the regions are particularly useful in diagnostic and therapeutic applications because of their effect upon receptor- ligand interaction. Methods for the production of polyclonal and monoclonal antibodies are well known, see for example Chap. 11 of Ausubel et al. (supra).

This invention also provides pharmaceutical compositions comprising an effective amount of antibody directed against the novel 5-HT₂ receptor to block binding of the naturally occurring ligands to that receptor in order to treat or ameliorate disease states associated with receptor activation. In its diagnostic embodiment the 5-HT₂ receptor on cell surfaces can be detected by contacting receptor bearing cells with antibodies of this invention and measuring the antibody/receptor complex. When the antibody is labeled with an analytically detectable reagent such a radioactivity, fluorescence, or an enzyme, the antibody can be use to detect the presence or absence of the receptor and/or its quantitative level.

Transgenic, non-human, animals may be obtained by transfecting appropriate fertilized eggs or embryos of a host with nucleic acids encoding the novel 5-HT₂ receptor disclosed herein, see for example U.S.Patents 4,736,866; 5,175,385; 5,175,384 and 5,175,386. The resultant transgenic animal may be used as a model for the study of receptor ligand interaction. Particularly, useful transgenic animals are those which display a detectable phenotype associated with the expression of the receptor. Drugs may then be screened for their ability to reverse or exacerbate the relevant phenotype. This invention also contemplates operatively linking the receptor coding gene to regulatory elements which are differentially responsive to various temperature or metabolic conditions, thereby effectively turning on or off the phenotypic expression in response to those conditions.

Although not necessarily limiting of this invention, following are some experimental data illustrative of this invention.

### Example 1 - Isolation of the novel human 5-HT₂ receptor.

### (a) Preparation of 5-HT_{2b} probe.

The cDNA sequence of the rat 5-HT_{2b} gene (1992 Embo. J. 11, 3481-3487) and the genomic structure of the mouse equivalent (Neuroreport 3, 345-348, 1992) have been described. Using this information, oligonucleotides A + B were designed to PCR amplify an approximately 396 bp fragment of exon 3.

oligonucleotide A: 5′ CGTAACTTTAGCTCTGTGTGATTCC

oligonucleotide B: 5′ CTTGGTCTTCTACTTTGTCACC

PCR reactions on 1 µg of human genomic DNA were set up in a total reaction volume of 100 µl containing 1 x PARR buffer from Advanced Biotechnologies Ltd (10 mM Tris pH 8, 1.5 mM MgCl₂, 0.1 mg/ml gelatin and proprietary ingredients), 0.2 mM dATP, dGTP, dTTP, dCTP, 1 µg each of oligonucleotides A and B. An initial cycle of 94°C-7', 45°C-1', 72°C-1' was run with 5 units of T3 DNA polymerase (Advanced Biotechnologies Ltd) being added at 45°C. This was followed by 30 cycles of 94°C-1', 45°C-1', 72°C-1' with an extension step on the final cycle of 5'. The amplification was carried out in a Techne programmable Dri-block PHC-1.

A 10 µl aliquot of the PCR product was run on a 3% TBE agarose gel containing ethidium bromide @ 1 µg/ml and a band of approximately 400 bp (C) was visualised. The remainder of the PCR reaction was run on a 3% TAE agarose gel, C was excised and the DNA eluted from the agarose into a final volume of 15 µl of TE buffer using a Geneclean II kit (Bio101) according to the manufacturer's directions.

7 µl of C was blunt ended and kinased using an adaptation of the manufacturer's directions in the Amersham blunting kit (Cat No RPN 1508) as follows. To 7 µl of C was added 1 µl 10 x blunting buffer, 0.5 µl 10 mM rATP, 1 µl T4 DNA polymerase, 0.5 µl T4 polynucleotide kinase (Gibco). The mix was incubated for 30' @ 37°C followed by a 15' incubation @ 65°C to inactivate the enzymes. The treated fragment was then ligated into the holding vector pUC19 which had been cut with SmaI and treated with BAP (cut vector supplied by Pharmacia Cat No:- 27-4860-01) as follows, 8 µl of fragment C and 5 ng of SmaI cut pUC19 were ligated together using reagents from the Amersham blunting kit (RPN 1508) according to the manufacturer's directions.

The resulting plasmid (vector D) was transformed to the bacterial host JM101 (using standard techniques described by Sambrook, J *et al* in Molecular Cloning: A Laboratory Manual). The inserted sequence was determined using Sanger dideoxy termination methodology and Sequenase Version 1 (USB).

The resulting 396 bp of DNA sequence showed an 84.9% homology to the published rat cDNA sequence in the region between nucleotides 1255 and 1652.

### (b) Tissue localisation studies: PCR, RT-PCR and nested PCR analaysis of human cortex, amygdala, hippocampus, stomach and small intestine

Although the rodent 5-HT_{2b} receptor is pharmacologically well characterised very little information has been available on its tissue distribution. Loric *et al* (Febs vol 312 number 2, 3 203-207) showed expression of the murine 5-HT_{2b} receptor in brain, heart and intestine while Foguet *et al* (Embo J. 11(9) 3841-3847) could only demonstrate expression in the stomach fundus of rat.

### (i) Brain samples

Total RNA was prepared from dissected human cortex, amygdala, hippocampus and whole rat brain using urea-lithium chloride, phenol chloroform extraction and ethanol/acetate precipitation. Poly A⁺ RNA was isolated using Dynabeads (Dynal Ltd.) according to the manufacturer's instructions. 1 µg of mRNA was reverse transcribed at 42°C for 60 mins in a total volume of 10 µl containing 20 mM Tris HCI pH 8.4, 50 mM KCl, 2.5 mM MgCl₂, 1 mM dNTP's (Pharmacia), 0.5 µM oligonucleotide B, 12.5 units RNAsin (Promega), 10 units AMV reverse transcriptase (Pharmacia). The RNA was hydrolysed in the presence of 5mM EDTA, 50 mM NaOH at 65°C for 60 mins then neutralised with HCI. The volume was increased to 100 µl with H₂O and the products were used immediately for PCR as follows: 10 µl of the reverse transcribed product was included in a 50 µl reaction containing 10 mM Tris pH 8.3, 50 mM KCl, 5mM MgCl₂, 200 µM of each dNTP, 1µM each of oligonucleotides A + B and 1.25 units of Amplitaq (Perkin Elmer). The reaction was overlaid with mineral oil and an initial cycle was run of 94°C-7', 55°C-1' and 72°C-2'. This was followed by thirty rounds of 94°C-1', 55° - 30 secs, 72°C-1'. 10 µl of this reaction mix was then subjected to a further 30 cycles of nested PCR using the same reaction conditions and substituting oligonucleotide E : 5' GGGTAGGCTACGTTTCCTCGGG and oligonucleotide F : 5' AAGACTGAATGGTTGAACTTCG for oligonucleotides A and B. A 5 µl aliquot of the PCR product was run on a 1% TBE agarose gel containing ethidium bromide at 1 µg/ml. A band of approximately 270 bp was visualised. The PCR product from the amygdala sample was gel purified and 7 µl was subcloned to SmaI cut pUC19 and partially sequenced as described above. The resulting 150bp of sequence was identical to a portion of product C.

### (ii) Stomach and small intestine

PCR reactions were performed on 10 ng samples of QuickClone ™ cDNA samples (Clontech) derived from human stomach (Cat. No: - 7126-1) and small intestine (Cat.No: ∼ 7176-1) under the same conditions as those described for the preparation of a 5HT2b probe. 10 µl aliquots of the PCR products were run on a 3% TBE agarose gel containing 1 µg/ml ethidium bromide and a band of approximately 400 bp in size was clearly visualised. The PCR product from the small intestine sample was subcloned and sequenced as described previously and was found to be identical in sequence to that isolated from human genomic DNA (product C).

### (c) Screening cDNA libraries

A commercially available λGT10 cDNA library derived from human small intestine (Clontech) was screened as described below using vector D as a probe. The library was plated and plaque lifts were performed as described by Sambrook J *et al,* in Molecular Cloning: A Laboratory Manual. The filters were hybridised in 6 x SSC, 10 x Denhardt's, 0.25% SDS, 100 µg/ml salmon sperm DNA at 65°C overnight and washed to a stringency of 0.5 x SSC 0.1% SDS for 30' @ 65°C x 2. Following autoradiography (-70°C with an intensifying screen for 16 hours) areas corresponding to positive signals were picked and subjected to several rounds of plaque purification. Clone 8.2 was isolated from this screen.

### (d) Analysis of clone 8.2

The λ phage isolate was found to contain a 1430 bp insert (Seq. ID. No. 1). Sequence analysis of the clone revealed it to be a novel human gene belonging to the G protein coupled receptor family having strong homology to the rat and mouse 5-HT_{2b} receptor sequences. Sequence analysis of the clone also revealed a 1197 bp open reading frame specifying a protein fragment of 399 amino acids and did not include a stop codon. The rat and mouse 5-HT_{2b} genes contain 479 and 504 amino acids respectively. A hydropathy analysis (Goldman, Engelman and Steitz) of the predicted protein shows seven hydrophobic domains, a key motif shared by all members of this receptor family. There are five potential sites for N linked glycosylation and consensus signals for phosphorylation are conserved between rat, mouse and this human sequence in the putative 1C3 loop region. A comparison of the predicted amino acid sequence of 8.2 (Seq. ID. No. 2) with other G protein coupled receptors shows that it shares strongest homology with the 5-HT₂ receptor family which indicates that it is appropriate to refer to 8.2 as a novel member of this family. Within this group 8.2 is most closely related to the rat and mouse 5-HT_{2b} genes (81.2% and 82.2% respectively).

Clone 8.2 may be used in its entirety as a radiolabelled probe to screen cDNA libraries derived from other tissues known to express the receptor, for example human stomach or brain cDNA libraries.

An alternative approach would be to generate a short probe directed at the 3' end of the receptor by digestion of the cloned DNA with the restriction enzymes EcoNI (cleaves at nucleotide 571 in the coding region) and Sty 1 (cleaves at nucleotide 975 in the coding region). Such a fragment would encompass the EC2 loop, TM5 and the IC3 loop. This may be radiolabelled and used to screen a human genomic library.

Any clones containing the remainder of the receptor coding region may be spliced to 8.2 to form a complete protein coding sequence.

The complete cDNA may be expressed in tissue culture systems as described in Example 2 to provide membranes for use in ligand binding assay or whole cell systems for use in second messenger assays.

### (e) Preparation of a 3' probe

The 1430bp EcoR1 insert from the λ phage isolate from (d) can be subcloned into EcoR1 cut pUC19 to form vector G. Fragment H, a short probe derived from the 3' end of clone 8.2 was obtained by digesting vector G with the restriction enzymes EcoNI (cleaves at nucleotide 575 in the ORF) and StyI (cleaves at nucleotide 975 in the ORF).

### (f) Screening a human genomic DNA library

Fragment H was labeled according to the manufacturer's instructions using the Amersham Megaprime system and used to screen a commercially available EMBL 3 genomic library derived from human leukocyte DNA (Clontech). The library was plated and plaque lifts were performed as described by Sambrook J. et al in Molecular Cloning: A Laboratory Manual. The filters were hybridized and washed under the same conditions as those described for screening the cDNA library (Example 1c). Following autoradiography (-70°C with an intensifying screen for 16 hours) areas corresponding to duplicated signals were picked and subjected to several rounds of plaque purification. Clone 8G was isolated from this screen.

### (g) Analysis of clone 8G

The EMBL3 phage isolate was found to contain an insert of >13kb in size. The DNA was cleaved with AccI, southern blotted as described by Sambrook J. et al in Molecular Cloning: A Laboratory Manual and probed sequentially with fragment H and vector D. A band of approximately 1.4kb in size was identified which hybridised only to vector D. This was blunt ended and subcloned into SmaI but pUC19 to form vector J. Analysis showed this fragment to contain DNA sequences found within clone 8.2 (from nucleotide 1136 in the coding region to 1197) and extending a further 249bp to a TAG stop codon. The ORF derived by overlapping clones 8.2 and 8G is 1443bp in length (Seq. ID No. 3) and encodes a protein of 481 amino acids (Seq. ID. No. 4). A hydropathy analysis (Goldman Engelman and Steitz) of the predicted protein shows seven hydrophobic domains, a key motif shared by all members of the G protein coupled receptor family. A comparison of the predicted amino acid sequence with other G-protein coupled receptors shows that it shares strongest homology with the 5HT-2 receptor group which indicates that it is appropriate to refer to this receptor as a novel member of that family. Within the group it is most closely related to the rat and mouse 5-HT_{2b} proteins (80.4% and 81.3% respectively across the protein coding region). There are five potential sites for N linked glycosylation and consensus sequences for phosphorylation are conserved between the rat and mouse 5-HT_{2b} genes and this human sequence in the putative IC3 loop region.

### (h) Assembly of the complete protein coding region

The complete coding region was assembled by ligating restriction fragments derived from clones 8.2 and 8G. A restriction fragment of approximately 1.4kb was isolated from vector G by digestion under standard conditions with EcoRI and BspMI. A restriction fragment of approximately 900bp was isolated from vector J by digestion with BglII and KpnI. These were ligated together into EcoRI/KpnI digested pUC19 via a linker, K, to form vector L.

Oligonucleotides which form linker K

5' ACCGGGCCACAAAGTCAGTAAAAACTCTCAGAAAACGCTCCAGTAA

5' GATCTTACTGGAGCGTTTTCTGAGAGTTTTTACTGACTTTGTGGCC

The orientation of the composite clone in pUC19 was altered by isolating the inserted DNA by cleaving with the enzymes XbaI and KpnI and religating into XbaI/KpnI cut pUC19 to form vector M.

### (i) Removal of 3' untranslated sequences

The 3' untranslated sequences are not required for protein production and may interfere with subcloning to a suitable eukaryotic expression vector. A fragment extending to a point 57bp beyond the TAG stop codon was made using PCR on human genomic DNA. Oligonucleotides N and P were used to PCR the fragment.

Oligonucleotide N: 5' TATAACTTTAGTTTTATGTGATTCC

Oligonucleotide P: 5' CTCATCATTATGTTTGATGACAACTGCC

PCR reactions were performed on 1 µg of human genomic DNA under the same conditions as those described for the preparation of a 5HT2b probe. A 10µl aliquot of the PCR product was run on a 3% gel and a band (Q) of approximately 420bp was visualised. The band was isolated and subcloned into Smal cut pUC18 as described in example 1(a). The resulting vector (R) was transformed to the bacterial host JM101. The insert was oriented with the 3' end of the gene adjacent to the BamHI site in the pUC18 polylinker. The inserted sequence was confirmed as identical to that seen in the genomic clone 8G over the same region.

### (j) Transfer of complete coding region to a eukaryotic expression vector

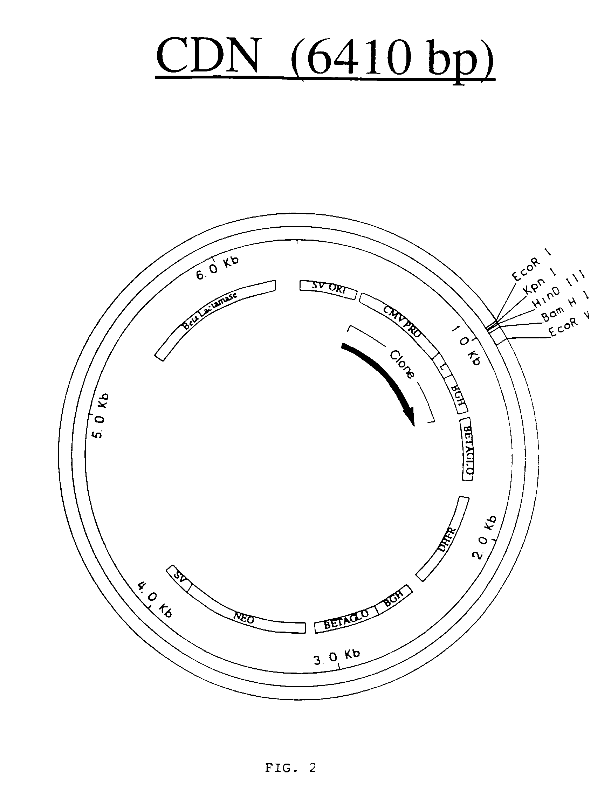

A 1500bp HindIII/EcoR1 fragment (contains 5' untranslated sequences and most of the protein coding region) from vector M was ligated along with a 200bp EcoRl/BamHl fragment (contains the remaining protein coding region) from vector R into the eukaryotic expression vector CDN (Fig. 2) which had been cut with HindIII/BamH1 to form vector S. The vector utilises the CMV promoter to drive high level expression in transfected cells. In addition it encodes a neomycin resistance gene to select for transfectants on the basis of ability to grow in G418 and a dihydrofolate reductase gene which permits amplification of the transfected gene in DHFR- cells.

### Example 2 - Heterologous Expression

### (a) Expression of partial receptor sequence

As the partial receptor sequence contains seven putative transmembrane domains it may prove possible to express it in a tissue culture system to provide membranes for use in ligand binding assays.

This may be effected by digestion of a holding vector such as pUC19 with clone 8.2 carried in the Smal site with EcoRI and BspMI. The resulting approximately 1430 bp fragment may be ligated along with linker H

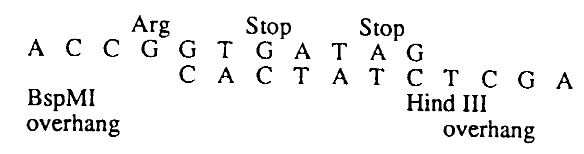

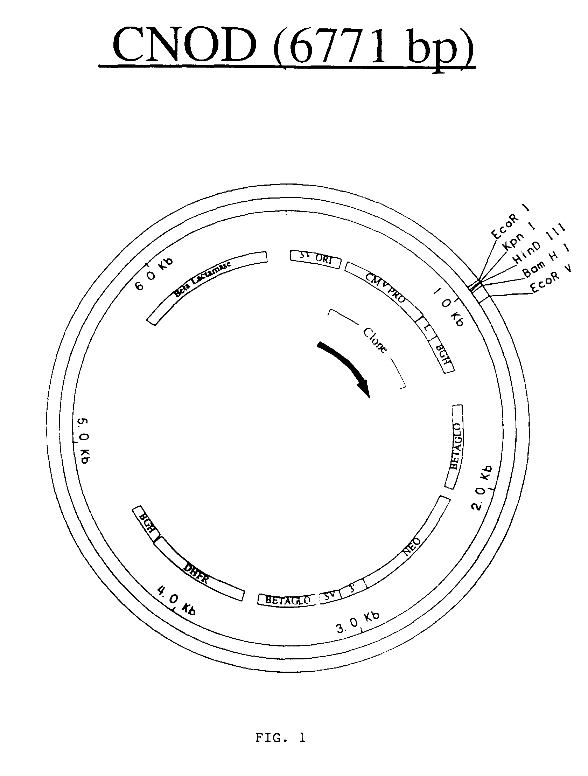

which contains two stop codons into the vector CNOD (Fig. 1) digested with EcoRI and Hind III to form vector J. The vector CNOD utilises the CMV promoter to drive high level expression in trasfected cells. In addition it encodes a neomycin resistance gene to select for transfectants on the basis of ability to grow in G418 and a dihydrofolate reductase gene which permits amplification of the transfected gene in DHFR⁻ cells. Such a vector should direct the production of protein in such host cell lines as CHO ACC98, a non adherent, DHFR⁻ cell line adapted to grow in serum free medium and human embryonic kidney 293 cells (ATCC CRL 1573). Transfected cell lines can be selected by G 418 resistance. Successful cell lines would express at least 5x10⁴-10⁵ receptor sites/cell and show binding of known ligands such as 5-HT, rauwolscine and ketanserin.

### (b) Expression of full length receptor sequence

The receptor has been expressed in human embryonic kidney 293 cells (ATCC CRL 1573) to provide membranes for use in ligand binding assays and whole cells for use in coupled assay systems.

Cells were seeded into 12 well plates at 30% confluence 24 hours prior to transfection. 2.5µg/well of vector S was used to form a calcium phosphate precipitate. This was added to the cells and incubated overnight at 37°C in an incubator gassed at 3% CO₂. The medium on the monolayer was then changed for Minimum Essential Medium with Earle's salts and L glutamine. After a further 24 hours incubation G418 (Geneticin) was added to a concentration of 800mg/ml of medium. The colonies which formed in each well were expanded as mass cultures for initial pharmacological characterisation.

Expression may alternatively be effected by subcloning the coding sequence for the receptor into a suitable expression vector such as pcDNA3 (Invitrogen) which uses the CMV promoter to drive high level expression in transfected mammalian cells. In addition, it encodes a neomycin resistance gene to select for stable transfectants on the basis of ability to grow in the presence of G418. Such a vector should direct the production of protein in other suitable host cell lines such as SKN-S-H human neuroblastoma cells (ATCC HTB II). Successful cell lines would express at least 5 x 10⁴ - 5 x 10⁵ receptor sites/cell and show binding of ligands such as 5-HT, rauwolscine and ketanserin.

### Example 3 - Compound Screening

To identify novel, potent agonists/antagonists of the novel 5-HT₂ receptor compound screening is performed on whole cells, cell homogenates or cell membranes isolated from cell lines expressing the recombinant receptor from Example 2. Affinity of a given compound for the receptor may be determined from its ability to inhibit binding of radioactively labeled compounds (e.g., ³H-5-HT or [¹²⁵I-DOI]) to prepared membranes using binding assays such as those described by Wainscott, D.B., et al., Mol. Pharmacol. 43:419-426 and Loric, S. et al., FEBS, 312:203-207. This method may be useful as an initial screen to identify selective compounds with measurable equilibrium dissociation constants at the novel 5-HT₂ receptor.

The potency of compounds may be assessed by measurement of an increase in metabolic activity which results in an acidification of extracellular fluid surrounding cells expressing the novel 5-HT₂ receptor. Acidification responses of the transformed human embryonic kidney 293 cell of Example 2(b) to maximally stimulating concentrations of 5-HT, as measured by the cytosensor microphysiometer (Molecular Devices), are clear (typically ≥ 10% above basal acidification rates). No such responses to 5-HT are detectable in parental cell lines. The affinity, potency and intrinsic efficacy of ligands may be measured by the ability of a ligand to evoke an acidification response, or attenuate the acidification response induced by 5-HT.

As the novel 5-HT₂ receptor is G-protein coupled the activity of compounds may also be assessed by measurement of guanine nucleotide exchange in manner similar to that described by Lazereno & Birdsall (1993) Br. J. Pharmacol., 109, 1120-1127, or by measurement of their ability to stimulate phospholipase C and generate polyphosphoinositides or to attenuate such stimulation since the receptor is believed to couple with reasonable efficiency to this second messenger system. In other systems, the receptor may be found to couple with some efficiency to other second messenger systems e.g. adenylyl cyclase.

Candidate compounds are of potential use in the treatment or prophylaxis of CNS, cardiovascular and gastrointestinal disorders involving 5-HT such as those described in Goodman and Gilman's The Pharmacological Basis of Therapeutics' 7th ED p. 629-631 and EP0189002, e.g. anxiety, depression, pathologies associated with blood brain or blood retinal barrier function, migraine, anorexia, obsessive compulsive disorders, Alzheimer's disease, sleep disorders, bulemia, panic attacks, epilepsy, withdrawal from drugs of abuse, schizophrenia, and are tested in appropriate animal models for these therapeutic indications.

**In the figures**
Fig. 1 is a diagram of the vector CNOD
Fig. 2 is a diagram of the vector CDN

### Sequence listing

### Seq. ID. No.1

Partial Nucleotide Sequence

ATGGCTCTCTCTTACAGAGTGTCTGAACTTCAAAGCACAATTCCTGAGCACATTTTG
CAGAGCACCTTTGTTCACGTTATCTCTTCTAACTGGTCTGGATTACAGACAGAATCA
ATACCAGAGGAAATGAAACAGATTGTTGAGGAACAGGGAAATAAACTGCACTGGGCA
GCTCTTCTGATACTCATGGTGATAATACCCACAATTGGTGGAAATACCCTTGTTATT
CTGGCTGTTTCACTGGAGAAGAAGCTGCAGTATGCTACTAATTACTTTCTAATGTCC
TTGGCGGTGGCTGATTTGCTGGTTGGATTGTTTGTGATGCCAATTGCCCTCTTGACA
ATAATGTTTGAGGCTATGTGGCCCCTCCCACTTGTTCTATGTCCTGCCTGGTTATTT
CTTGACGTTCTCTTTTCAACCGCATCCATCATGCATCTCTGTGCCATTTCAGTGGAT
CGTTACATAGCCATCAAAAAGCCAATCCAGGCCAATCAATATAACTCACGGGCTACA
GCATTCATCAAGATTACAGTGGTGTGGTTAATTTCAATAGGCATTGCCATTCCAGTC
CCTATTAAAGGATAGAGACTGATGTGGACAACCCAAACAATATCACTTGTGTGCTG
ACAAAGGAACGTTTTGGCGATTTCATGCTCTTTGGCTCACTGGCTGCCTTCTTCACA
CCTCTTGCAATTATGATTGTCACCTACTTTCTCACTATCCATGCTTTACAGAAGAAG
GCTTACTTAGTCAAAAACAAGCCACCTCAACGCCTAACATGGTTGACTGTGTCTACA
GTTTTCCAAAGGGATGAAACACCTTGCTCGTCACCGGAAAAGGTGGCAATGCTGGAT
GGTTCTCGAAAGGACAAGGCTCTGCCCAACTCAGGTGATGAAACACTTATGCGAAGA
ACATCCACAATTGGGAAAAAGTCAGTGCAGACCATTTCCAACGAACAGAGAGCCTCA
AAGGTCCTAGGGATTGTGTTTTTCCTCTTTTTGCTTATGTGGTGTCCCTTCTTTATT
ACAAATATAACTTTAGTTTTATGTGATTCCTGTAACCAAACTACTCTCCAAATGCTC
CTGGAGATATTTGTGTGGATAGGCTATGTTTCCTCAGGAGTGAATCCTTTGGTCTAC
ACCCTCTTCAATAAGACATTTCGGGATGCATTTGGCCGATATATCACCTGCAATTAC

### Seq. ID. No.2

Partial Peptide Sequence

MALSYRVSELQSTIPEHILQSTFVHVISSNWSGLQTESIPEEMKQIVEEQGNKLHWA
ALLILMVIIPTIGGNTLVILAVSLEKKLQYATNYFLMSLAVADLLVGLFVMPIALLT
IMFEAMWPLPLVLCPAWLFLDVLFSTASIMHLCAISVDRYIAIKKPIQANQYNSRAT
AFIKITVVWLISIGIAIPVPIKGIETDVDNPNNITCVLTKERFGDFMLFGSLAAFFT
PLAIMIVTYFLTIHALQKKAYLVKNKPPQRLTWLTVSTVFQRDETPCSSPEKVAMLD

GSRKDKALPNSGDETLMRRTSTIGKKSVQTISNEQRASKVLGIVFFLFLLMWCPFFI
TNITLVLCDSCNQTTLQMLLEIFVWIGYVSSGVNPLVYTLFNKTFRDAFGRYITCNY

### Seq. ID. No.3

Full Nucleotide Sequence

ATGGCTCTCTCTTACAGAGTGTCTGAACTTCAAAGCACAATTCCTGAGCACATTTTG
CAGAGCACCTTTGTTCACGTTATCTCTTCTAACTGGTCTGGATTACAGACAGAATCA
ATACCAGAGGAAATGAAACAGATTGTTGAGGAACAGGGAAATAAACTGCACTGGGCA
GCTCTTCTGATACTCATGGTGATAATACCCACAATTGGTGGAAATACCCTTGTTATT
CTGGCTGTTTCACTGGAGAAGAAGCTGCAGTATGCTACTAATTACTTTCTAATGTCC
TTGGCGGTGGCTGATTTGCTGGTTGGATTGTTTGTGATGCCAATTGCCCTCTTGACA
ATAATGTTTGAGGCTATGTGGCCCCTCCCACTTGTTCTATGTCCTGCCTGGTTATTT
CTTGACGTTCTCTTTTCAACCGCATCCATCATGCATCTCTGTGCCATTTCAGTGGAT
CGTTACATAGCCATCAAAAAGCCAATCCAGGCCAATCAATATAACTCACGGGCTACA
GCATTCATCAAGATTACAGTGGTGTGGTTAATTTCAATAGGCATTGCCATTCCAGTC
CCTATTAAAGGATAGAGACTGATGTGGACAACCCAAACAATATCACTTGTGTGCTG
ACAAAGGAACGTTTTGGCGATTTCATGCTCTTTGGCTCACTGGCTGCCTTCTTCACA
CCTCTTGCAATTATGATTGTCACCTACTTTCTCACTATCCATGCTTTACAGAAGAAG
GCTTACTTAGTCAAAAACAAGCCACCTCAACGCCTAACATGGTTGACTGTGTCTACA
GTTTTCCAAAGGGATGAAACACCTTGCTCGTCACCGGAAAAGGTGGCAATGCTGGAT
GGTTCTCGAAAGGACAAGGCTCTGCCCAACTCAGGTGATGAAACACTTATGCGAAGA
ACATCCACAATTGGGAAAAAGTCAGTGCAGACCATTTCCAACGAACAGAGAGCCTCA
AAGGTCCTAGGGATTGTGTTTTTCCTCTTTTTGCTTATGTGGTGTCCCTTCTTTATT
ACAAATATAACTTTAGTTTTATGTGATTCCTGTAACCAAACTACTCTCCAAATGCTC
CTGGAGATATTTGTGTGGATAGGCTATGTTTCCTCAGGAGTGAATCCTTTGGTCTAC
ACCCTCTTCAATAAGACATTTCGGGATGCATTTGGCCGATATATCACCTGCAATTAC
CGGGCCACAAAGTCAGTAAAAACTCTCAGAAAACGCTCCAGTAAGATCTACTTCCGG
AATCCAATGGCAGAGAACTCTAAGTTTTTCAAGAAACATGGAATTCGAAATGGGATT
AACCCTGCCATGTACCAGAGTCCAATGAGGCTCCGAAGTTCAACCATTCAGTCTTCA
TCAATCATTCTACTAGATACGCTTCTCCTCACTGAAAATGAAGGTGACAAAACTGAA
GAGCAAGTTAGTTATGTATAG

### Seq. ID. No.4

Full Peptide Sequence

MALSYRVSELQSTIPEHILQSTFVHVISSNWSGLQTESIPEEMKQIVEEQGNKLHWA
ALLILMVIIPTIGGNTLVILAVSLEKKLQYATNYFLMSLAVADLLVGLFVMPIALLT
IMFEAMWPLPLVLCPAWLFLDVLFSTASIMHLCAISVDRYIAIKKPIQANQYNSRAT
AFIKITVVWLISIGIAIPVPIKGIETDVDNPNNITCVLTKERFGDFMLFGSLAAFFT
PLAIMIVTYFLTIHALQKKAYLVKNKPPQRLTWLTVSTVFQRDETPCSSPEKVAMLD

GSRKDKALPNSGDETLMRRTSTIGKKSVQTISNEQRASKVLGIVFFLFLLMWCPFFI
TNITLVLCDSCNQTTLQMLLEIFVWIGYVSSGVNPLVYTLFNKTFRDAFGRYITCNY
RATKSVKTLRKRSSKIYFRNPMAENSKFFKKHGIRNGINPAMYQSPMRLRSSTIQSS
SIILLDTLLLTENEGDKTEEQVSYV

### Seq. ID. No.5 (oligonucleotide A)

CGTAACTTTAGCTCTGTGTGATTCC

### Seq. ID. No.6 (oligonucleotide B)

CTTGGTCTTCTACTTTGTCACC

### Seq. ID. No.7 (oligonucleotide E)

GGGTAGGCTACGTTTCCTCGGG

### Seq. ID. No.8 (oligonucleotide F)

AAGACTGAATGGTTGAACTTCG

### Seq. ID. No.9 (Linker K)

ACCGGGCCACAAAGTCAGTAAAAACTCTCAGAAAACGCTCCAGTAA

### Seq. ID. No.10 (Linker K)

GATCTTACTGGAGCGTTTTCTGAGAGTTTTTACTGACTTTGTGGCC

### Seq. ID. No.11 (Oligonucleotide N)

TATAACTTTAGTTTTATGTGATTCC

### Seq. ID. No.12 (Oligonucleotide P)

CTCATCATTATGTTTGATGACAACTGCC

### Seq. ID. No.13 (Linker H)

ACCGGTGATAG

### Seq. ID. No.14 (Linker H)

CACTATCTCGA

## Claims

1. An isolated nucleic acid molecule selected from the group consisting of:
(a) a nucleic acid molecule comprising the DNA sequence shown in Seq. ID. No. 1 or Seq. ID No. 3 and
(b) a nucleic acid molecule differing from the nucleic acid molecule of (a) in codon sequence due to the degeneracy of the genetic code but encoding the same protein.

2. RNA having the sequence corresponding to the DNA of claim 1.

3. A vector comprising the nucleic acid molecule of claim 1 or 2.

4. A recombinant host cell comprising the vector of claim 3.

5. An antisense oligonucleotide having a sequence which is capable of binding to the nucleic acid molecule of claim 2.

6. An isolated protein which is a novel human 5-HT₂ receptor encoded by a nucleic acid molecule of claim 1.

7. An isolated protein which is a novel human 5-HT₂ receptor defined by the amino acid sequence listed in Seq. ID. No. 2 or 4 or variants thereof with a substantially homologous amino acid sequence having at least an 85% match with, but retaining the receptor binding activity of, the novel 5-HT₂ receptor.

8. A novel 5-HT₂ receptor according to claim 7 comprising the amino acid sequence listed in Seq. ID. No. 2.

9. A novel 5-HT₂ receptor according to claim 7 having the amino acid sequence listed in Seq. ID. No. 4.

10. A process for preparing the receptor of any one of claims 6 to 9 which comprises expressing DNA encoding said receptor and recovering the expression product.

11. An antibody directed to the receptor of any one of claims 6 to 9.

12. The antibody according to claim 11 which is a monoclonal antibody.

13. A method of screening compounds to identify those compounds which bind to the receptor of any one of claims 6 to 9, comprising: providing a recombinant host cell expressing on the surface thereof said receptor, said receptor being associated with a second component capable of providing a detectable signal in response to the binding of a compound to said receptor; contacting a plurality of candidate compounds with said host cells under conditions sufficient to permit binding of compounds to the receptor; and identifying those compounds capable of receptor binding by detecting the signal produced by said second component.

14. A method for diagnosing a 5-HT₂ receptor according to any one of claims 6 to 9 on cell surfaces comprising contacting receptor bearing cells with the antibody of claim 11 and measuring the antibody-receptor complex.

15. Use of a pharmaceutical composition containing the antibody of claim 11 in the preparation of a medicament for treating disease states associated with excess 5-HT₂ receptor activation which composition is effective to block binding of naturally occurring ligands to said receptor and thereby alleviating said disease states.

## Patentansprüche

1. Isoliertes Nucleinsäuremolekül, ausgewählt aus (a) einem Nucleinsäuremolekül, das die in Sequenz ID-Nr. 1 oder Sequenz ID-Nr. 3 gezeigte DNA-Sequenz umfaßt, und (b) einem Nucleinsäuremolekül, das aufgrund der Degeneration des genetischen Codes von dem Nucleinsäuremolekül aus (a) in der Codonsequenz unterschiedlich ist, jedoch für das gleiche Protein codiert.

2. RNA mit der der DNA gemäß Anspruch 1 entsprechenden Sequenz.

3. Vektor, der das Nucleinsäuremolekül gemäß Anspruch 1 oder 2 umfaßt.

4. Rekombinante Wirtszelle, die den Vektor gemäß Anspruch 3 umfaßt.

5. Antisense-Oligonucleotid mit einer Sequenz, die in der Lage ist, an das Nucleinsäuremolekül gemäß Anspruch 2 zu binden.

6. Isoliertes Protein, das ein neuer Human 5-HT₂ Rezeptor ist, der durch ein Nucleinsäuremolekül gemäß Anspruch 1 codiert wird.

7. Isoliertes Protein, das ein neuer Human 5-HT₂ Rezeptor ist, der definiert ist durch die Aminosäuresequenz, die in Sequenz-ID Nr. 2 oder 4 aufgelistet ist, oder Varianten davon mit einer im wesentlichen homologen Aminosäuresequenz, die mindestens 85%-ige Übereinstimmung mit dem neuen 5-HT₂ Rezeptor aufweist und dessen Rezeptorbindungsaktivität beibehält.

8. Neuer 5-HT₂ Rezeptor gemäß Anspruch 7, der die Aminosäuresequenz gemäß Sequenz-ID Nr. 2 umfaßt.

9. Neuer 5-HT₂ Rezeptor gemäß Anspruch 7, der die in Sequenz-ID Nr. 4 aufgelistete Aminosäuresequenz aufweist.

10. Verfahren zur Herstellung des Rezeptors gemäß mindestens einem der Ansprüche 6 bis 9, umfassend die Exprimierung der für den Rezeptor codierenden DNA und Abtrennung des Exprimierungsprodukts.

11. Antikörper, der gegen den Rezeptor gemäß mindestens einen der Ansprüche 6 bis 9 gerichtet ist.

12. Antikörper gemäß Anspruch 11, der ein monoklonaler Antikörper ist.

13. Verfahren zum Screenen von Verbindungen, wodurch solche Verbindungen identifiziert werden, die an den Rezeptor gemäß mindestens einen der Ansprüche 6 bis 9 binden, das folgendes umfaßt: Bereitstellung einer rekombinanten Wirtszelle, die auf ihrer Oberfläche den besagten Rezeptor exprimiert, der Rezeptor ist mit einer zweiten Komponente assoziiert, die in der Lage ist, als Antwort auf die Bindung einer Verbindung an den Rezeptor ein nachweisbares Signal zu liefern; Kontaktierung einer Mehrzahl in Frage kommender Verbindungen mit den Wirtszellen unter Bedingungen, die geeignet sind, die Bindung der Verbindungen an den Rezeptor zu erlauben; und Identifizierung derjenigen Verbindungen, die in der Lage sind, an den Rezeptor zu binden, indem das durch die besagte zweite Komponente erzeugte Signal nachgewiesen wird.

14. Verfahren zur Diagnostizierung eines 5-HT₂ Rezeptors gemäß mindestens einem der Ansprüche 6 bis 9 auf Zelloberflächen, umfassend die Kontaktierung von rezeptortragenden Zellen mit dem Antikörper gemäß Anspruch 11 und Messung des Antikörperrezeptorkomplexes.

15. Verwendung einer pharmazeutischen Zusammensetzung, die den Antikörper gemäß Anspruch 11 enthält, zur Herstellung eines Medikaments zur Behandlung von Krankheitszuständen, die mit erhöhter 5-HT₂ Rezeptor-Aktivierung assoziiert sind, wobei die Zusammensetzung wirksam ist zur Blockierung der Bindung natürlich auftretender Liganden an den Rezeptor, wodurch die besagten Krankheitszustände gelindert werden.

## Revendications

1. Molécule d'acide nucléique isolée choisie dans le groupe consistant en :
(a) une molécule d'acide nucléique comprenant la séquence d'ADN représentée dans la Seq. ID. n° 1 ou la Seq.ID N° 3, et
(b) une molécule d'acide nucléique différente de la molécule d'acide nucléique de (a) par la séquence de codons en raison de la dégénérescence du code génétique, mais codant pour la même protéine.

2. ARN ayant la séquence correspondant à l'ADN suivant la revendication 1.

3. Vecteur comprenant la molécule d'acide nucléique suivant la revendication 1 ou 2.

4. Cellule hôte recombinante comprenant le vecteur suivant la revendication 3.

5. Oligonucléotide antisens ayant une séquence qui est capable de se lier à la molécule d'acide nucléique suivant la revendication 2.

6. Protéine isolée qui est un récepteur 5-HT₂ humain nouveau codé par une molécule d'acide nucléique suivant la revendication 1.

7. Protéine isolée qui est un récepteur 5-HT₂ humain nouveau défini par la séquence d'aminoacides énumérés dans la Seq.ID. N° 2 ou 4 ou ses variants ayant une séquence d'aminoacides essentiellement homologue présentant une concordance d'au moins 85 % avec le, mais conservant l'activité de liaison du récepteur 5-HT₂ nouveau.

8. Récepteur 5-HT₂ nouveau suivant la revendication 7, comprenant la séquence d'aminoacides indiquée dans la Seq.ID. N° 2.

9. Récepteur 5-HT₂ nouveau suivant la revendication 7, ayant la séquence d'aminoacides indiquée dans la Seq. ID. N° 4.

10. Procédé pour la préparation du récepteur suivant l'une quelconque des revendications 6 à 9, qui comprend l'étape consistant en l'expression de l'ADN codant pour ledit récepteur et l'étape consistant à recueillir le produit d'expression.

11. Anticorps dirigé contre le récepteur suivant l'une quelconque des revendications 6 à 9.

12. Anticorps suivant la revendication 11, qui est un anticorps monoclonal.

13. Procédé pour sélectionner des composés afin d'identifier les composés qui se lient au récepteur suivant l'une quelconque des revendications 6 à 9, comprenant les étapes consistant :
à prendre une cellule hôte recombinante exprimant sur sa surface ledit récepteur, ledit récepteur étant associé à un second constituant capable d'engendrer un signal détectable en réponse à la liaison d'un composé audit récepteur, à mettre en contact une pluralité de composés candidats avec lesdites cellules hôtes dans des conditions suffisantes pour permettre la liaison des composés au récepteur ; et à identifier les composés aptes à la liaison au récepteur par détection du signal produit par ledit second constituant.

14. Méthode pour diagnostiquer un récepteur 5-HT₂ suivant l'une quelconque des revendications 6 à 9 sur les surfaces de cellules, comprenant la mise en contact de cellules portant le récepteur avec l'anticorps suivant la revendication 11 et le dosage du complexe anticorps-récepteur.

15. Utilisation d'une composition pharmaceutique contenant l'anticorps suivant la revendication 11 dans la préparation d'un médicament destiné au traitement d'états pathologiques associés à un excès d'activation des récepteurs 5-HT₂, composition qui est efficace pour bloquer la liaison de ligands naturels audit récepteur et soulager ainsi lesdits états pathologiques.
